# EUROPEAN PATENT SPECIFICATION

(11) **EP 0 626 965 B1**
(45) Date of publication and mention of the grant of the patent: **01.07.1998**
(21) Application number: 93903973.1
(22) Date of filing: 16.02.1993
(51) Int. Cl.: C07D 475/04

(54) **PROCESS FOR SEPARATING STEREOISOMERS OF FOLINIC ACID**
VERFAHREN ZUR TRENNUNG VON STEREOISOMERE VON FOLINSÄURE
PROCEDE DE SEPARATION DE STEREOISOMERES DE L'ACIDE FOLINIQUE

(30) Priority: 20.02.1992 IT MI920367
(43) Date of publication of application: 07.12.1994
(73) Proprietor: BRACCO S.p.A., 20134 Milano (IT)
(72) Inventor: RIPA, Giorgio, I-20131 Milano (IT); PIVA, Rodolfo, I-20129 Milano (IT); FELDER, Ernst, CH-6826 Riva S. Vitale (CH)
(74) Representative: Bianchetti, Giuseppe, Prof.
(86) International application number: EP9300361
(87) International publication number: WO9317022

(56) References cited:
- EP-A- 0 266 042
- EP-A- 0 348 641
- EP-A- 0 356 934
- EP-A- 0 367 902
- EP-A- 0 432 441
- EP-A- 0 455 013
- EP-A- 0 495 204
- WO-A-88/08844
- WO-A-91/13890
- US-A- 2 688 018

## Description

The present invention relates to a process for separating stereoisomers of folinic acid.

Folinic acid, i.e. N-(5-formyl-(6R,S)-5,6,7,8-tetrahydropteroyl)-L-glutamic acid, when obtained by chemical synthesis, is formed by an equimolar mixture of its two (6R) and (6S) diastereomeric forms.

It is known that only the (6S) isomer, as calcium salt, has the well-known pharmacological activity of the product, while the other one is totally devoid of it. Therefore, there is a strong need for a process allowing the preparation of the optically pure (6S) form.

Various attempts were made to synthetize (6S) isomer through asymmetrical synthesis but they were not fully successful from the industrial point of view.

As far as the separation of both diastereomers from their equimolar mixture is concerned, it is worth citing US patent 2688018 and patent application WO 88/03844, even if they have not yet provided the ideal solution to the problem.

EP-A-455013 discloses ammonium salts of N5-methyl-5,6,7,8-tetrahydrofolic acid which can be used for the separation of the (6R) or (6S) diastereoisomers.

It has now been found a process allowing the separation and the isolation with good yields of the two optically pure (6R) and (6S) forms of folinic acid, both free and salified with alkaline-earth metal ions, from the equimolar diastereomeric mixture produced by chemical synthesis starting from folic acid.

Another remarkable embodiment of this invention relates to a process for the preparation and isolation of salt derivatives of (6R)-folinic acid, respectively (6S)-folinic acid, characterised by an excellent optical purity (O.P.), with suitable at least dibasic organic amines, which are aliphatic, straight, cyclic and/or heterocyclic, from said diastereomeric equimolar mixture.

Another remarkable embodiment of this invention is represented by the new salts of (6R)-folinic and/or (6S)-folinic acid with said amines.

Folinic acid is usually prepared by synthesis starting from folic acid, which contains a (S) chiral centre in that part of the molecule corresponding to (S)-glutamic acid. By hydrogenating the double bond between the 5-and 6- positions of the pterinic residue, a new chiral centre in the 6- position is originated with a (6R,S) configuration. The subsequent formylation step of nitrogen atom at the 5- position leads to the production of (6R,S)-folinic acid.

It now has been surprisingly found that, by crystallization of (6R,S)-folinic acid salts with at least dibasic organic amines from a suitable solvent or, preferably, from binary or ternary mixtures of solvents, mixtures of said salts enriched in their (6R) or (6S) form can be obtained, according to the amines used.

Said optically enriched mixtures can be further enriched in the preponderant isomeric form through one or more recrystallizations to obtain the corresponding salts with said amines of (6R)-folinic acid, respectively (6S)-folinic acid, with an O.P. of at least 99%, usually higher than 99%.

The conversion from these salts to the corresponding salts with alkaline-earth metal ions with the same optical purity is very easy.

By way of example, from the mixture enriched in its (6S)-form, the calcium salt (or the corresponding salts with alkaline-earth metal ions) of (6S)-folinic acid can be prepared with an O.P. higher than 99%. This result can preferably be achieved according to one of the following methods:
a) from the aqueous solution of the (6S) enriched diamino folinate, through treatment with an alkaline-earth metal chloride at neutral pH and optionally in presence of a precipitating solvent, the alkaline-earth metal salt of the (6S) enriched folinic acid is separated. Further crystallizations from a suitable solvent or solvent mixtures lead to the optically pure (6S) form of the desired alkaline-earth metal folinate,
b) the (6S) enriched diamino folinate is recrystallized from suitable solvent mixtures in order to obtain the optically pure (6S) form. The corresponding salt with alkaline-earth metal ions is subsequently obtained through exchange, as described in a).

The same process can be applied to the (6R) enriched mixture to obtain the corresponding (6R) isomer with an O.P. higher than 99%.

Preferred metal cations to salify (6R,S)-folinic acid and its optically pure diastereomeric forms are those of the alkaline-earth metals, especially Ca, Mg, Sr, Ba. Ca²⁺ ion is particularly preferred.

The process of the invention comprises the steps of:
a) reacting racemic folinic acid, optionally obtained from its salts with an alkaline-earth metal by acidification with mineral acids at pH 1-3 at 0-25°C, in a liquid medium containing at least one component selected from dipolar aprotic organic solvents, water and water-soluble protic organic solvents, with a stoichiometric amount or a molar excess not higher than 20% of a diamine of general formulae from I to III 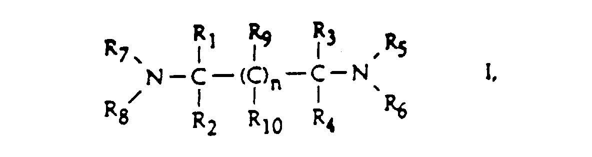 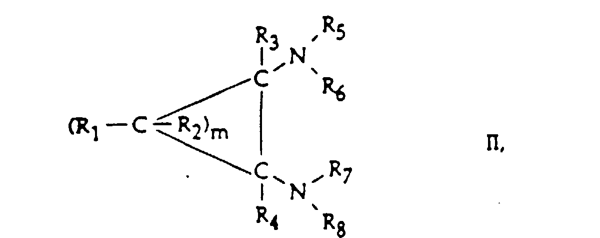 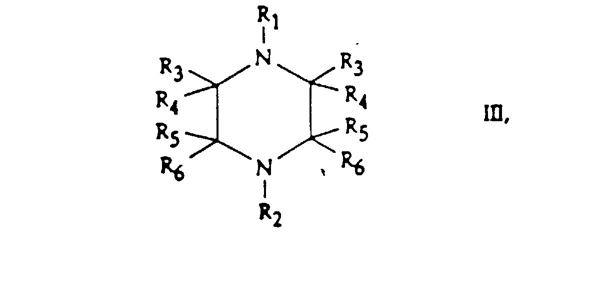 wherein:
   R₁ to R₈ which are the same or different, are H or straight or branched C₁₋₆ alkyl groups, which can be substituted by 1-4 OH groups and/or by 1-4 alkoxy or hydroxyalkoxy groups,
   R₉ to R₁₀ which are the same or different, are R₁ or OH,
   n 0-6
   m 2-8
   or of straight or macrocyclic polyamines comprising from 3 to 6 N atoms to give the corresponding racemic folinic diamino or polyamines salt;
b) crystallizing said diamino-salt from its solution in a mixture of water and a water-soluble dipolar aprotic organic solvent, optionally containing an organic water-soluble protic solvent, wherein a first solid which separates upon cooling contains a major portion of a first one of the said (6R) or (6S;) diastereomers, the major portion of the other second isomer remaining in the crystallization mother-liquors;
c) purifying by recrystallizing once or more times the isomer-enriched solid form step (b), from the same solvent mixture used in this step, until the first diastereomeric diamino-salt is obtained in a desired optical purity, and optionally converting this salt into the corresponding alkaline-earth metal salt;
d) isolating the solid remaining dissolved in the mother-liquors form step (b), through precipitation by diluting the solution with a dipolar aprotic solvent and/or a protic organic solvent, wherein the solid that is obtained mainly contains the second isomer, and recrystallizing this solid as recited in step c), or treating said mother-liquors, after evaporation of the organic solvents, with an excess of water-soluble alkaline-earth metal mineral salts and recrystallizing once or more times the obtained alkaline-earth metal salt of said second isomer of folinic acid with water until the desired optical purity is reached.

Preferably in step (a) an alkaline-earth metal salt of racemic folinic acid is used, wherein said alkaline-earth metal cation is insolubilized by reaction with a suitable anion and filtered off.

The racemic diamino-salt of folinic acid is isolated in step (a) by precipitation or crystallization, treating the reaction mixture with a water-soluble protic organic solvent.

The optical purity degree for both isomers which result from crystallization in steps (c) or (d) is 99% or more.

The invention also provides a process for the separation and the purification of the two diastereomeric forms, (6R) and (6S), of folinic acid and/or its salts with alkaline-earth metal ions or with at least dibasic organic amines, up to an optical purity (O.P.) level higher than 99%, starting from a racemic mixture of the same, this process comprising the following steps:
a) preparing the diamino salts of said racemic mixture by reacting racemic free folinic acid, dissolved in a dipolar aprotic organic solvent or in a mixture of the same with a protic organic solvent, or suspended in water, with the amines of claim 1,
   or preparing said diamino salts by reacting an aqueous solution of a racemic alkaline-earth metal folinate with a mineral acid at pH 1-3 at 0-25°C and then with said amines and eliminating said metal ions by formation of insoluble compounds of the same and filtration thereof, wherein the obtained racemic mixture of said diamino salts crystallizes from the reaction medium or is precipitated from the same by adding a water-soluble protic organic solvent or by diluting said reaction medium in said water-soluble protic organic solvent,
b) crystallizing said precipitated racemic mixture form a solution thereof in a solvent made of water and a water-soluble dipolar aprotic organic solvent optionally containing a protic organic solvent, wherein the solid that separates upon crystallization contains a major portion of one of the two (6R) or (6S) diastereomeric forms of the diamino salt, this form being less soluble than the other one, which remains largely dissolved in crystallization mother liquors,
c) recrystallizing once or more times the isomer-enriched solid obtained under step b), from the same kind of solvent used in this step, to give the desired diamino (6R) or (6S)-folinate with an O.P. higher than 99%, wherein said optically pure diamino folinate can be subsequently transformed into the corresponding alkaline-earth metal salts of the same optical purity by reaction with an excess of water-soluble alkaline-earth metal mineral salts in aqueous medium,
d) precipitating the other isomer-enriched diamino salt from crystallization mother liquors, obtained under step b), by dilution with a dipolar aprotic solvent and/or a protic organic solvent, collecting the solid thereof and recrystallizing it once or more times under the same conditions as in step c), wherein said other stereoisomeric form of diamino-folinate is obtained with an O.P. higher than 99%, the corresponding alkaline-earth metal salts being then optionally obtained with the same optical purity by reaction with an excess of water-soluble alkaline-earth metal mineral salts,
   or directly treating said mother liquors of step b), after elimination of any organic solvent, with an excess of water-soluble alkaline-earth metals mineral salt, wherein the obtained isomer-enriched alkaline-earth metal folinates are recrystallized once or more times from water to give the corresponding metal folipates with an O.P. higher than 99%.

In step a) of the above process, the reaction of racemic folinic acid with said amines is preferably performed in a dipolar aprotic organic solvent, or in a mixture of the same with a protic organic solvent, or in water, in suspension phase, by subsequently diluting the resulting solution with or in a water-soluble organic protic solvent.

More preferably during step a), the reaction of said racemic alkaline-earth metal folinates with said amines is performed in water in the presence of inorganic or organic anions, which form water-insoluble derivatives with said alkaline-earth metal cations, being said resulting insoluble compounds preferably sulphates or oxalates.

The crystallization of said salts of folinic acid is performed according to steps b) or c), by dissolving them in said solvent mixtures at a temperature ranging from 25 to 70°C, preferably from 30 to 50°C and then by gradually cooling the resulting solution to 25-0°C, preferably to 20-0°C.

The optically pure (6R) or (6S) diamino folinates, obtained under step c), are transformed into the corresponding alkaline-earth metal salts of the same optical purity in water, in presence of water-soluble mineral salts of said metals, preferably chlorides or nitrates, in a weight ratio (w/w) ranging from 0.2 to 5, preferably 2 to 4, parts of said metal salt per part of said diamino salt, at a temperature ranging from 0 to 35°C, preferably 5 to 25°C, by maintaining pH between 6 and 7, preferably by adding 1N sodium hydroxide.

The optically pure salts of folinic acid, obtained under steps c) or d), are dissolved in water and the resulting aqueous solutions are acidified by adding a mineral acid to a pH value of 1-3 at a temperature ranging from 0 to 25°C, wherein the corresponding (6R) or (6S) isomers of free folinic acid precipitate with an O.P. higher than 99%.

By way of non-limiting example, particularly preferred diamines can be selected from: ethylenediamine, 1,2-diamino-propane, 1,3-diamino-propane, 1,3-diamino-2-hydroxy-propane, (cis)-1,2-diamino-cyclohexane, (trans)-1,2-diamino-cyclohexane, piperazine, 1,4-dimethyl-piperazine, 2-methyl-piperazine, 2,5-dimethyl-piperazine. Equally preferred amines can also be polyamino straight derivatives or macrocyclic compounds containing from 3 to 6 N atoms.

By way of example, among these last ones, 1,4,7,10-tetraazacyclododecane and its derivatives substituted both on ethylene bridges and on nitrogen atoms can be cited.

Suitable solvents for the crystallization of diastereomeric mixtures of folinic acid salts with said amines are preferably binary or ternary water/dipolar aprotic organic solvent mixtures and/or mixtures of water/dipolar aprotic organic solvent/protic organic solvent.

Preferred protic organic solvents are, i.e., methanol, ethanol, n-propanol, isopropanol, ethylene glycol, 1,2-propylene glycol, 1,3-propylene glycol, formamide, N-methyl-formamide.

Preferred dipolar aprotic organic solvents are, i.e., dimethylformamide (DMF), dimethylacetamide (DMAC), dimethylsulphoxide (DMSO), N-methyl-pyrrolydone (NMP), hexamethylphosphoramide (HMPT).

The process of this invention is hereinafter described, employing, by way of non-limiting example, as starting material the calcium salt of (6R,S)-folinic acid and it is performed according to the following steps:

### Step 1

Precipitation of (6R,S)-folinic acid from an aqueous solution of its calcium salt, by adjusting pH between 1 and 3 with mineral acids at a temperature ranging from 0 to 25°C.

### Step 2

Formation of (6R,S)-folinic acid salts with diamines/polyamines in a dipolar aprotic organic solvent, preferably DMAC. Said salts are obtained under a solid form by:
a) dissolution of (6R,S)-folinic acid in a dipolar aprotic solvent, in a weight/volume ratio ranging from 1:5 to 1:20 (w/v);
b) reaction, at a temperature from 5 to 35°C, with a stoichiometric amount, or a molar excess not higher than 20%, of the desired amine, dissolved either in the same dipolar aprotic solvent in a ratio ranging from 1:5 to 1:50 (w/v) or in a protic organic solvent in a ratio ranging from 1:5 (w/v) to 1:15 (w/v);
c) gradually cooling the reaction medium at a temperature ranging from 25°C to 5°C, up to a complete precipitation of the desired salt. Precipitation can be easened by adding a protic organic solvent in a volume ratio ranging from 0.1:1 to 2:1 (v/v) compared with the dipolar aprotic solvent.

Said salification reaction can also be performed in water, in suspension phase, followed by dilution of the resulting aqueous solution with or in a water-soluble protic organic solvent, which acts as a precipitating agent.

### Step 3

Partial separation of folinic acid diastereomers by crystallization or insolubilization of its salts with said amines from a water/dipolar aprotic water-soluble solvent, optionally with addition of an organic protic solvent.

The weight/volume (w/v) ratio salt/mixtures of solvents can range from 1:2 to 1:60, preferably from 1:4 to 1:45.

The composition of the mixture water/dipolar aprotic solvent (v/v) can range from 1:0.5 to 1:20, preferably from 1:1 to 1:15; such mixture can also contain a protic organic solvent in amounts up to 70% in volume, preferably to 50%, in case said protic organic solvent is needed.

The salts of (6R,S)-folinic acid with the above mentioned amines can directly be dissolved in the desired mixture of solvents at a temperature comprised between 25 and 70°C, preferably 30 and 50°C.

On the other hand, they can also be dissolved in water. The desired amount of dipolar aprotic solvent, optionally together with the required additional amount of protic organic solvent, is subsequently added to said aqueous solution.

Then these solutions are gradually cooled to nearly 0°C while crystallization takes place.

The obtained solid moiety consists of a mixture of (6R) and (6S) isomers of amino-folinates, which is enriched in the less soluble form (i.e. R or S, depending on the amino compound used as salifying agent).

For example, crystallization of ethylenediamino (6R,S)-folinate leads to a mixture enriched in the (6R) isomer salt.

On the contrary, from piperazino (6R,S)-folinate, the (6S) form preferably crystallizes.

Usually said enriched solid mixtures show an O.P. ranging from 65 to 98%.

Crystallization mother liquors are obviously enriched in the other isomeric form and show an O.P. value usually ranging from 65 to 80%.

### Step 4

Further purification of the partially optically purified amino folinates, deriving from Step 3, is performed through one or more recrystallizations of the same from a mixture water/dipolar aprotic solvent whose composition ranges from 1:1 (v/v) to 1:20 (v/v). This crystallizing mixture can optionally contain also a protic organic solvent in amounts up to 50% in volume. The desired optically pure amino folinate is obtained with an O.P. higher than 99%.

Then said optically pure amino folinate can be transformed into the corresponding (6R), respectively (6S), calcium folinate.

One of the preferred ways consists in crystallizing said amino folinate from water (pH = 7 and temperature in the range 0-25°C) in the presence of 0.2 to 5, preferably 2 to 4, parts (w/w) of water soluble calcium salts, preferably chlorides or nitrates.

Alternatively the desired optically pure calcium folinate can be precipitated from the above mentioned aqueous Ca²⁺ containing solution by adding a great excess of a protic water-soluble organic solvent.

### Step 5

The stereoisomers of folinic acid, which are contained in the mother liquors resulting from the crystallizations described in Step 3, are also recovered from said mother liquors with an O.P. higher than 99%.

They can be obtained either as amino or calcium salts, preferably following one of the procedures hereinafter described.
a) From the above mentioned mother liquors the organic protic solvent, if present, is distilled off under reduced pressure at temperatures from 25 to 50°C and subsequently the water-soluble dipolar aprotic solvent is removed by extracting the mixture with a water-insoluble organic solvent, preferably methylene chloride (CH₂Cl₂). To the resulting aqueous solution CaCl₂ is added in amounts corresponding to 0.2-5, preferably to 2-4, parts in weight (w/w) in relation with the starting quantity of amino (6R,S)-folinate. Then pH is adjusted to a value ranging between 6 and 7 by means of 1N NaOH and, by adding ethanol the precipitation of the desired calcium salt is obtained, operating at a temperature of 0-25°C, preferably 5-25°C.
   Calcium folinate, enriched in its prevailing diastereomer, with an O.P. ranging from 65 to 85%, is recovered through filtration. The optically pure calcium folinate (O.P. higher than 99%) is finally obtained through one or more crystallizations from 10 to 100 volume of water, with pH ranging from 6 to 7 and at temperatures from 0 to 25°C.
b) To mother liquors, deriving from Step 3, suitable precipitating solvents, either dipolar aprotic or protic organic solvents, are added in amounts equal to 0.05-5 volumes in relation with the mother liquors volume. By cooling to 0-20°C, the prevailing optical isomer of diamino folinate can be preferentially crystallized with an O.P. higher than 70%.
   The desired amine folinate with an O.P. higher than 99% is then obtained by one or more crystallizations from water/dipolar aprotic solvent mixtures with a ratio ranging from 1:1 to 1:20 (v/v), optionally by adding a protic organic solvent, in amounts up to 50% in volume.

Amino (6R,S)-folinate obtained as described in Steps 1 and 2 can alternatively be prepared directly from a calcium (6R,S)-folinate aqueous solution. To this solution another aqueous solution can be added containing, in stoichiometric amounts compared with the starting folinate, the desired amine salified with a mineral or organic acid which forms insoluble salts with alkaline-earth metal cations, preferably anions of sulphuric or oxalic acid. The insoluble salt precipitating, i.e. calcium oxalate, is filtered off and to the resulting aqueous solution, containing the desired diamino (6R,S)-folinate, the suitable amount of dipolar aprotic solvent is subsequently added, optionally added with protic organic solvent as previously described.

Crystallization can be performed at a temperature ranging from 0 to 25°C and lasts from 1 h up to five days by operating at a neutral or slightly alkaline pH. The procedure described in step 1 can also be adopted in order to obtain the optically pure isomers of free folinic acid (6R or 6S), with an O.P. higher than 99%, starting from the corresponding optically pure salts of the same obtained according to step 4 and 5.

The following examples aim at better disclosing the most remarkable features of this invention.

### EXAMPLE 1

Preparation of pure (6R,S)-folinic acid.

100 g of calcium (6R,S)-folinate pentahydrate, dissolved in 750 ml of water and 670 ml of 0.5 N hydrochloric acid, are added dropwise at the same time to 300 ml of water, under stirring and at 5-20°C. Then the obtained suspension, containing (6R,S)-folinic acid, is kept under stirring for 1 h at 5-25°C. After that the precipitate is filtered, washed with water and dried. 76 g of (6R,S)-folinic acid are obtained.
- HPLC titre:: ≥ 99% performed under the following chromatographic conditions:
- Column:: Hibar^{R} Lichrosphere RP18-5 µm (Merck): 250 mm length; 4 mm diameter.
- Mobile phase:: A = phosphate buffer pH 7.8;
B = 25% methanol in phosphate buffer pH
7.8 (v/v).
- Flow rate:: 1.2 ml/min; column temperature: 35°C; UV 254 nm detector.
- Gradient:: from 0 to 2 min B = 12%, from 2 to 20 min straight gradient up to B = 80% then maintenance for 3 min, then inverse gradient from 23 to 35 min up to B = 12%.

Following the same procedure the following free acid compounds have been obtained:
- (6S)-folinic acid with an O.P. higher than 99%, starting from calcium (6S)-folinate of Example 5.
- (6R)-folinic acid with an O.P. higher than 99%, starting from ethylenediamino (6R)-folinate of Example 6.

### EXAMPLE 2

Preparation of ethylenediamino (6R,S)-folinate. 75 g of (6R,S)-folinic acid are dissolved in 750 ml of dimethylacetamide (DMAC). 10.5 g of ethylenediamine, dissolved in 400 ml of the same solvent, are added at a temperature of 5-35°C. The precipitated salt suspension is kept under stirring for about 1 h, at 5-25°C, then is filtered. After drying, 82 g of ethylenediamino (6R,S)-folinate are obtained.
- HPLC titre:: ≥ 98.5% according to the chromatographic method of Example 1.

### EXAMPLE 3

Diastereomer separation of ethylenediamino (6R,S)-folinate.

80 g of ethylenediamino (6R,S)-folinate are dissolved in 740 ml of water. 920 ml of DMAC are added under stirring at a temperature of 25-40°C. Then temperature is lowered to 5°C and the product crystallizes, under stirring, for 24 h. After that the precipitate is filtered off and washed with ethanol. After drying, 40.2 g of ethylenediamino folinate are obtained, containing the 80% of (6R) diasteroisomer. The (6R) form content is determined by performing a chiral HPLC under the following conditions:
- Column:: length 250 mm; diameter 4 mm; manually fed with Chiral Si 300 BSA (SERVA), 5 µm stationary phase.
- Mobile phase:: 0.25 M solution of NaH₂PO₄ in water.
- Flow rate:: 1.0 ml/min; column temperature 40°C; detector: UV 280 nm (310 nm).

Mother liquors, after filtration, contain ethylenediamino (6S)-folinate with a 60% diastereomeric excess (determined with chiral HPLC under the above mentioned conditions).

Chromatographic conditions disclosed in Examples 1 and 3 are also used for controls and determinations in the remaining examples.

### EXAMPLE 4

Ethylenediamino (6S)-folinate isolation and purification.

To the mother liquors derived from the crystallization described in Example 3 and mostly containing ethylenediamino (6S)-folinate, with a 60% diastereomeric excess, 170 ml of DMAC are added. The solution is kept under stirring for 48 h at 10°C. The resulting precipitate is filtered, washed with ethanol and dried. 15 g of ethylenediamino (6S)-folinate are obtained with an O.P. higher than 99% (chiral HPLC control was performed according to Example 3).
- HPLC titre:: ≥ 98.5; [α]²⁰ _{D} = -14°8° (c= 2.1% H₂O).

### EXAMPLE 5

Preparation of calcium (6S)-folinate from ethylenediamino (6S)-folinate.

To 10 g of ethylenediamino (6S)-folinate (obtained as disclosed in Example 4), dissolved into 100 ml of water, 10 g of calcium chloride and 400 ml of ethanol are added under stirring at 5-25°C. The separated precipitate is filtered, washed with aqueous ethanol (95%) and dried. Then, the product is dissolved again in water at pH 6-6.5, at 50-60°C. One part in weight of calcium chloride is added and pH is adjusted to 7 with NaOH 1N. The desired compound crystallizes at 0-25°C for 16 h.

The precipitated crystalline product is filtered, washed with cold water and aqueous ethanol and dried. 11 g of calcium (6S)-folinate containing about 20% of crystallization water are obtained with an O.P. higher than 99% (through chiral HPLC).
- HPLC titre:: > 99%; [α]²⁰ _{D} = -15° (c= 1% H₂O).

### EXAMPLE 6

Purification of ethylenediamino (6R)-folinate.

35 g of ethylenediamino (6R)-folinate (obtained as described in Example 3), with 80% O.P., are recrystallized from 500 ml of water/DMAC with a volumetric ratio of 1/1.25. Temperature and crystallization time are the following: 0-25°C and 48h. The crystallized product is filtered, washed with ethanol and dried. 25 g of ethylenediamino (6R)-folinate with an O.P. higher than 99% (chiral HPLC) are obtained. [α]²⁰ _{D} = + 44.4° (c=2.1% H₂O).

### EXAMPLE 7

Calcium (6S)-folinate isolation and purification from an ethylenediamino folinate mixture enriched in the (6S) isomer.

The mother liquors deriving from the crystallization described in Example 3 and mostly containing ethylenediamino (6S)-folinate with a 60% diasteroisomeric excess are extracted with 5 x 400 ml methylene chloride. To the resulting aqueous solution, 50 g of calcium chloride are added under stirring at 0-25°C, then pH is adjusted to 7 with NaOH 1 N and 250 ml of ethanol are added. The solid separated product is filtered, washed with aqueous ethanol and dried. The resulting product is recrystallized twice from water, at pH 7, at 0-25°C and in the presence of 3 parts in weight of calcium chloride and washing the filtered product every time, the first time with cold water and then with ethanol.

17 g of calcium (6S)-folinate are obtained with an O.P. higher than 99% (through chiral HPLC) and with about 20% crystallization water content.
- HPLC titre:: > 99%; [α]²⁰ _{D} = - 15° (c=1% H₂O).

### EXAMPLE 8

Piperazino (6R,S)-folinate preparation.

80 g of (6R,S)-folinic acid, prepared as described in Example 1, are dissolved in 950 ml of DMAC. 17.5 g of piperazine dissolved into 2000 ml of ethanol are added thereto under stirring at a temperature of 5-30°C. The resulting suspension is kept under stirring for 3 h at room temperature and then filtered. The wet product is washed with ethanol, and then dried. 90.5 g of piperazino (6R,S)-folinate are obtained.
- HPLC titre:: > 99%.

### EXAMPLE 9

Diastereomer separation of piperazino (6R,S)-folinate.

85 g of piperazino (6R,S)-folinate are suspended in 3600 ml of a mixture water/DMAC (1:10.5 v/v). Temperature is raised to 35°C, until the solid compound is completely dissolved. Crystallization takes 3 days at 5°C, under stirring. The obtained precipitate is filtered, washed with ethanol and dried. 35 g of piperazino (6S)-folinate with an O.P. of ≥ 97% are recovered.

### EXAMPLE 10

Piperazino (6S)-folinate purification.

10 g of piperazino (6S)-folinate, obtained as described in Example 9, are recrystallized from a mixture water/DMAC (1:10.5 v/v) following the procedure of Example 6. After filtering, washing and drying the crystalline product, 8 g of piperazino (6S)-folinate with an O.P. higher than 99% (chiral HPCL control) are obtained.

### EXAMPLE 11

Piperazino (6R)-folinate isolation and purification.

To the mother liquors derived from the crystallization described in Example 9, mostly containing piperazino (6R)-folinate, 1800 ml of ethanol are added. Crystallization takes place at 5°C, for 64 h. The precipitate is then filtered, washed with ethanol and dried. 30 g of piperazino (6R)-folinate, with an O.P. of ≥ 94% (chiral HPCL control) are recovered.

Said salt is then recrystallized from a mixture water/DMAC/ethanol (1:10:5 v/v/v), following the procedure of Example 6, to give the desired product with an O.P. higher then 99%.

### EXAMPLE 12

Preparation of calcium (6S)-folinate from piperazino (6S)-folinate.

Calcium (6S)-folinate with an O.P. higher than 99% is obtained in yield of 80% from the piperazino (6S)-folinate deriving from Example 10 following the procedure described in Example 5.

### EXAMPLE 13

Preparation of 1,4-dimethylpiperazino (6R,S)-folinate 80 g of (6R,S)-folinic acid, prepared as described in Example 1, are dissolved into 800 ml of DMAC. 21 g of 1,4-dimethyl-piperazine, dissolved in 460 of DMAC, are added under stirring at a temperature of 5-25°C, then 700 ml of ethanol are added. The solution is kept under stirring for 5 days at 5°C to obtain a precipitate which is filtered, washed with ethanol and dried.

90 g of 1,4-dimethylpiperazine (6R,S)-folinate are obtained.
(HPLC titre: > 99%).

### EXAMPLE 14

Preparation of 1,3-diamino-2-hydroxy-propane (6R,S)-folinate.

80 g of (6R,S)-folinic acid, prepared as described in Example 1, are dissolved into 800 ml of DMAC. 16.8 g of 1,3-diamino-2-hydroxy-propane dissolved into 420 ml of DMAC are added under stirring at 5-25°C. The solution is kept under stirring for 1.5 h at the same temperature to obtain a precipitate which is filtered, washed first with DMAC and then with ethanol, and then dried. 89 g of 1,3-diamino-2-hydroxy-propane (6R,S)-folinate are obtained (HPLC titre > 99%).

### EXAMPLE 15

Partial separation of 1,3-diamino-2-hydroxy-propane (6R,S)-folinate diastereomers.

87 g of 1,3-diamino-2-hydroxy-propane (6R,S)-folinate are dissolved into 600 ml of water. 780 ml of DMAC are added under stirring and at a temperature of 25-40°C. The product crystallizes at 5°C from the solution kept under stirring for 2 days, then is filtered. The wet product is washed with ethanol. After drying, 50 g of 1,3-diamino-2-hydroxy-propane folinate containing about 70% of diastereomer (6R) (chiral HPLC), are obtained. In the residual crystallization mother liquors, the (6S) diastereomer is present in about 55% diastereomeric excess.

### EXAMPLE 16

1,3-diamino-2-hydroxy-propane (6S)-folinate isolation and purification.

160 ml of DMAC are added to the mother liquors deriving from the fractional crystallization described in Example 15, and containing 1,3-diamino-2-hydroxy-propane (6S)-folinate, with a 55% diastereomeric excess. The solution is kept under stirring for 30 h at 5°C to obtain a precipitate which is filtered and washed with ethanol. After drying, 20 g of 1,3-diamino-2-hydroxy-propane (6S)-folinate are obtained with 95% O.P. This product is recrystallized from 300 ml of water/DMAC mixture in a volumetric ratio of 1/1.5 at a 0-25°C temperature. The resulting precipitate is filtered and washed with ethanol. After drying, 16 g of 1,3-diamino-2-hydroxy-propane (6S)-folinate are obtained with an O.P. higher than 99% (determined through chiral HPLC), titre ≥ 98.5%.

## Claims

1. A process for the separation of the (6R) and (6S) diastereomers of folinic acid and the recovery thereof in free or salified form of desired high optical purity, which process comprises the steps of:
a) reacting racemic folinic acid, optionally obtained from its salt with an alkaline-earth metal by acidification with mineral acids at pH 1-3 at 0-25°C, in a liquid medium containing at least one component selected from dipolar aprotic organic solvents, water and water-soluble protic organic solvents, with a stoichiometric amount or a molar excess not higher than 20% of a diamine of general formulae from I to III 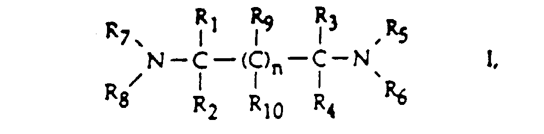 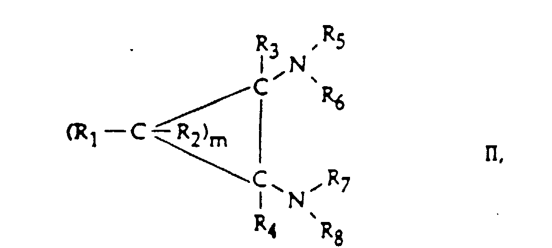 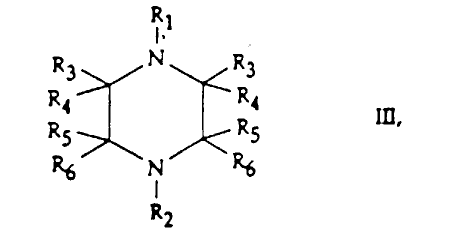 wherein:
R₁ to R₈ which are the same or different, are H or straight or branched C₁₋₆ alkyl groups, which can be substituted by 1-4 OH groups and/or by 1-4 alkoxy or hydroxyalkoxy groups,
R₉ to R₁₀ which are the same or different, are R₁ or OH,
n 0-6,
m 2-8.
or of straight or macrocyclic polyamines comprising from 3 to 6 N atoms to give the corresponding racemic folinic diamino or polyamines salt;
b) crystallizing said diamino-salt from its solution in a mixture of water and a water-soluble dipolar aprotic organic solvent, optionally containing an organic water-soluble protic solvent, wherein a first solid which separates upon cooling contains a major portion of a first one of the said (6R) or (6S) diastereomers, the major portion of the other second isomer remaining in the crystallization mother-liquors;
c) purifying by recrystallizing once or more times the isomer-enriched solid from step (b), from the same solvent mixture used in this step, until the first diastereomeric diamino-salt is obtained in a desired optical purity, and optionally converting this salt into the corresponding alkaline-earth metal salt;
d) isolating the solid remaining dissolved in the mother-liquors from step (b), through precipitation by diluting the solution with a dipolar aprotic solvent and/or a protic organic solvent, wherein the solid that is obtained mainly contains the second isomer, and recrystallizing this solid as recited in step c), or treating said mother-liquors, after evaporation of the organic solvents, with an excess, of water-soluble alkaline-earth metal mineral salts and recrystallizing once or more times the obtained alkaline-earth metal salt of said second isomer of folinic acid with water until the desired optical purity is reached.

2. The process of claim 1, in which in step (a) an alkaline-earth metal salt of racemic folinic acid is used, wherein said alkaline-earth metal cation is insolubilized by reaction with a suitable anion and filtered off.

3. The process of claim 1, in which the racemic diamino-salt of folinic acid is isolated in step (a) by precipitation or crystallization, treating the reaction mixture with a water-soluble protic organic solvent.

4. The process of claim 1, in which the desired optical purity degree for both isomers which result from crystallization in steps (c) or (d) is 99% or more.

5. The method of claim 1 for the separation and the purification of the two diastereomeric forms, (6R) and (6S), of folinic acid and/or its salts with alkaline-earth metal ions or with at least dibasic organic amines, up to an optical purity (O.P.) level higher than 99%, starting from a racemic mixture of the same, this process comprising the following steps:
a) preparing the diamino salts of said racemic mixture by reacting racemic free folinic acid, dissolved in a dipolar aprotic organic solvent or in a mixture of the same with a protic organic solvent, or suspended in water, with the amines of claim 1,
or preparing said diamino salts by reacting an aqueous solution of a racemic alkaline-earth metal folinate with a mineral acid at pH 1-3 at 0-25°C and then with said amines and eliminating said metal ions by formation of insoluble compounds of the same and filtration thereof, wherein the obtained racemic mixture of said diamino salts crystallizes from the reaction medium or is precipitated from the same by adding a water-soluble protic organic solvent or by diluting said reaction medium in said water-soluble protic organic solvent,
b) crystallizing said precipitated racemic mixture form a solution thereof in a solvent made of water and a water-soluble dipolar aprotic organic solvent optionally containing a protic organic solvent, wherein the solid that separates upon crystallization contains a major portion of one of the two (6R) or (6S) diastereomeric forms of the diamino salt, this form being less soluble than the other one, which remains largely dissolved in crystallization mother liquors,
c) recrystallizing once or more times the isomer-enriched solid obtained under step b), from the same kind of solvent used in this step, to give the desired diamino (6R) or (6S)-folinate with an O.P. higher than 99%, wherein said optically pure diamino folinate can be subsequently transformed into the corresponding alkaline-earth metal salts of the same optical purity by reaction with an excess of water-soluble alkaline-earth metal mineral salts in aqueous medium,
d) precipitating the other isomer-enriched diamino salt from crystallization mother liquors, obtained under step b), by dilution with a dipolar aprotic solvent and/or a protic organic solvent, collecting the solid thereof and recrystallizing it once or more times under the same conditions as in step c), wherein said other stereoisomeric form of diamino-folinate is obtained with an O.P. higher than 99%, the corresponding alkaline-earth metal salts being then optionally obtained with the same optical purity by reaction with an excess of water-soluble alkaline-earth metal mineral salts,
or directly treating said mother liquors of step b), after elimination of any organic solvent, with an excess of water-soluble alkaline-earth metals mineral salt, wherein the obtained isomer-enriched alkaline-earth metal folinates are recrystallized once or more times from water to give the corresponding metal folinates with an O.P. higher than 99%.

6. The method of claim 5, wherein, during step a), the reaction of racemic folinic acid with said amines is performed in a dipolar aprotic organic solvent, or in a mixture of the same with a protic organic solvent, or in water, in suspension phase, by subsequently diluting the resulting solution with or in a water-soluble organic protic solvent.

7. The method of claim 5, wherein, during step a), the reaction of said racemic alkaline-earth metal folinates with said amines is performed in water in the presence of inorganic or organic anions, which form water-insoluble derivatives with said alkaline-earth metal cations, being said resulting insoluble compounds preferably sulphates or oxalates.

8. The method of claim 5, wherein crystallizations under steps b) and c) are performed in a solvent mixture made of water and a water-soluble dipolar aprotic organic solvent, wherein said components range, in a volumetric ratio, from 1:0.5 (v/v) to 1:20 (v/v), or said crystallizations are performed in a solvent containing said water/water-soluble dipolar aprotic organic solvent mixture further added with a protic organic solvent in amounts up to 70% in volume.

9. The method of claim 5, wherein the weight/volume ratio between said racemic diamino folinate salts and the solvent mixture used for crystallization of the same, according to step b), ranges from 1:2 (w/v) to 1:60 (w/v).

10. The method of claim 5, wherein crystallization of said salts of folinic acid is performed according to steps b) or c), by dissolving them in said solvent mixtures at a temperature ranging from 25 to 70°C, and then by gradually cooling the resulting solution to 25-0°C.

11. The method of claim 5, wherein the optically pure (6R) or (6S) diamino folinates, obtained under step c), are transformed into the corresponding alkaline-earth metal salts of the same optical purity in water, in presence of water-soluble mineral salts of said metals in a weight ratio (w/w) ranging from 0.2 to 5, parts of said metal salt per part of said diamino salt, at a temperature ranging from 0 to 35°C, by maintaining pH between 6 and 7, preferably by adding 1N sodium hydroxide.

12. The method of claim 5, wherein preferred cations of alkaline-earth metal are selected from Ca²⁺, Mg²⁺, Sr²⁺, Ba²⁺.

13. The method of claim 12, wherein the preferred metal ion is Ca²⁺.

14. The method of anyone of claims 1-13, wherein the preferred amines are selected from: ethylenediamine, 1,2-diamino-propane, 1,3-diamino-propane, 1,3-diamino-2-hydroxy-propane, (cis)-1,2-diamino-cyclohexane, (trans)-1,2-diamino-cyclohexane, piperazine, 2-methylpiperazine, 1,4-dimethyl-piperazine, 2,5-dimethylpiperazine, 1,4,7,10-tetraazacyclododecane, derivatives and lower and higher homologous thereof.

15. The method of claim 5, wherein the preferred dipolar aprotic solvents are selected from: dimethylformamide, dimethylacetamide, dimethylsulphoxide, N-methyl-pyrrolydone, hexamethylphosphoramide.

16. The method of claim 5, wherein the preferred protic organic solvents are selected from: methanol, ethanol, n-propanol, isopropanol, ethylene glycol, 1,2-propylene glycol, 1,3-propylene glycol, formamide, N-methylformamide.

17. The method of claim 5, wherein the optically pure salts of folinic acid, obtained under steps c) or d), are dissolved in water and the resulting aqueous solutions are acidified by adding a mineral acid to a pH value of 1-3 at a temperature ranging from 0 to 25°C, wherein the corresponding (6R) or (6S) isomers of free folinic acid precipitate with an O.P. higher than 99%.

18. The method of claim 11, wherein said water-soluble mineral salts of alkaline-earth metals are selected from chlorides and nitrates.

19. Diamino (6R,S)-folinates wherein the salifying diamine is selected from: ethylenediamine, 1,2-diaminopropane, 1,3-diamino-propane, 1,3-diamino-2-hydroxypropane, (cis)-1,2-diamino-cyclohexane, (trans)-1,2-diamino-cyclohexane, piperazine, 2-methyl-piperazine, 1,4-dimethyl-piperazine, 2,5-dimethyl-piperazine.

20. Diamino (6R)-folinates with an O.P. higher than 99%, wherein the salifying diamine is selected from: ethylenediamine, 1,2-diamino-propane, 1,3-diaminopropane, 1,3-diamino-2-hydroxy-propane, (cis)-1,2-diamino-cyclohexane, (trans)-1,2-diamino-cyclohexane, piperazine, 2-methyl-piperazine, 1,4-dimethylpiperazine, 2,5-dimethyl-piperazine.

21. Diamino (6S)-folinates with an O.P. higher than 99%, wherein the salifying diamine is selected from: ethylenediamine, 1,2-diamino-propane, 1,3-diaminopropane, 1,3-diamino-2-hydroxy-propane, (cis)-1,2-diamino-cyclohexane, (trans)-1,2-diamino-cyclohexane, piperazine, 2-methyl-piperazine, 1,4-dimethylpiperazine, 2,5-dimethyl-piperazine.

22. A diamino-folinate selected from:
Ethylenediamino (6R,S)-folinate;
Piperazino (6R,S)-folinate;
1,4-dimethyl-piperazino (6R,S)-folinate;
1,3-diamino-2-hydroxy-propyl (6R,S)-folinate;
Ethylenediamino (6R)-folinate;
Ethylenediamino (6S)-folinate;
Piperazino (6R)-folinate;
Piperazino (6S)-fplinate;
1,4-dimethyl-piperazino (6R)-folinate;
1,4-dimethyl-piperazino (6S)-folinate;
1,3-diamino-2-hydroxy-propyl (6R)-folinate;
1,3-diamino-2-hydroxy-propyl (6S)-folinate.

## Patentansprüche

1. Verfahren zur Trennung der (6R)- und (6S)-Diastereomeren von Folinsäure und deren Wiedergewinnung in freier Form oder in Salzform mit der angestrebten hohen optischen Reinheit, umfassend die Stufen:
a) Umsetzung von racemischer Folinsäure, die gegebenenfalls aus ihrem Erdalkalimetallsalz durch Ansäuern mit Mineralsäuren bei pH 1-3 bei 0-25°C erhalten worden ist, in einem flüssigen Medium, enthaltend mindestens eine Komponente, ausgewählt aus dipolaren aprotischen organischen Lösungsmitteln, Wasser und wasserlöslichen protischen organischen Lösungsmitteln mit einer stöchiometrischen Menge oder einem molaren Überschuß von nicht höher als 20% eines Diamins der allgemeinen Formeln I bis III 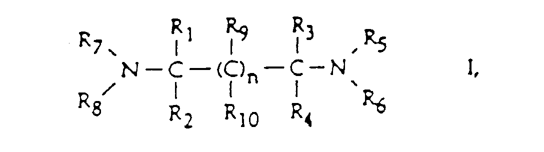 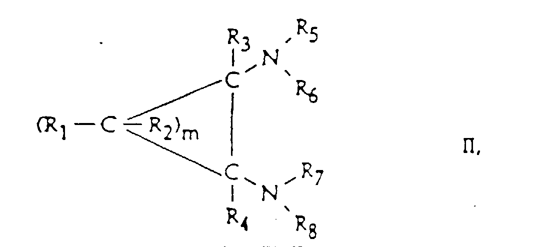 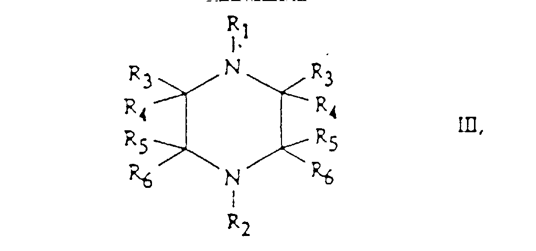 worin:
R₁ bis R₈, die gleich oder verschieden sind, für H oder geradkettige oder verzweigte C₁₋₆-Alkylgruppen stehen, die durch 1-4 OH-Gruppen und/oder durch 1-4 Alkoxy- oder Hydroxyalkoxygruppen substituiert sein können,
R₉ bis R₁₀, die gleich oder verschieden sind, R₁ oder OH sind,
n = 0-6,
m = 2-8,
oder von geradkettigen oder macrocyclischen Polyaminen mit 3 bis 6 N-Atomen, um das entsprechende racemische Diamino- oder Polyaminsalz der Folinsäure zu bilden;
b) Kristallisation des genannten Diaminosalzes aus seiner Lösung in einem Gemisch aus Wasser und einem wasserlöslichen dipolaren aprotischen organischen Lösungsmittel, das gegebenenfalls ein organisches wasserlösliches protisches Lösungsmittel enthält, wobei ein erster Feststoff, der sich nach dem Abkühlen abscheidet, einen Hauptteil eines ersten der genannten (6R)- oder (6S)-Diastereomeren enthält, und der Hauptteil des anderen, zweiten Isomeren in den Kristallisations-Mutterlaugen zurückbleibt;
c) Reinigung durch einmalige oder mehrmalige Umkristallisation des an dem Isomeren angereicherten Feststoffs der Stufe b) aus dem gleichen Lösungsmittelgemisch, wie in dieser Stufe verwendet, bis das erste diastereomere Diaminosalz mit der gewünschten optischen Reinheit erhalten wird, und gegebenenfalls Umwandlung dieses Salzes in das entsprechende Erdalkalimetallsalz;
d) Isolierung des zurückgebliebenen, in den Mutterlaugen der Stufe b) aufgelösten Feststoffs durch Ausfällen durch Verdünnung der Lösung mit einem dipolaren aprotischen Lösungsmittel und/oder einem protischen organischen Lösungsmittel, wobei der erhaltene Feststoff hauptsächlich das zweite Isomere enthält, und Umkristallisation dieses Feststoffs, wie in Stufe c) beschrieben, oder Behandlung der genannten Mutterlaugen nach Abdampfen der organischen Lösungsmittel mit einem Überschuß von wasserlöslichen Erdalkalimetall-Mineralsalzen und einmalige oder mehrmalige Umkristallisation des erhaltenen Erdalkalimetallsalzes des genannten zweiten Isomeren der Folinsäure mit Wasser, bis die gewünschte optische Reinheit erreicht wird.

2. Verfahren nach Anspruch 1, dadurch **gekennzeichnet,** daß in Stufe a) ein Erdalkalimetallsalz der racemischen Folinsäure eingesetzt wird, wobei das genannte Erdalkalimetallkation durch Umsetzung mit einem geeigneten Anion unlöslich gemacht wird und abfiltriert wird.

3. Verfahren nach Anspruch 1, dadurch **gekennzeichnet,** daß das racemische Diaminosalz der Folinsäure in Stufe a) durch Ausfällung oder Kristallisation isoliert wird, wobei das Reaktionsgemisch mit einem wasserlöslichen protischen organischen Lösungsmittel behandelt wird.

4. Verfahren nach Anspruch 1, dadurch **gekennzeichnet,** daß der angestrebte optische Reinheitsgrad für beide Isomere, die durch Kristallisation in den Stufen c) oder d) resultieren, 99% oder mehr ist.

5. Verfahren nach Anspruch 1 zur Trennung und Reinigung der zwei diastereomeren Formen (6R) und (6S) der Folinsäure und/oder ihrer Salze mit Erdalkalimetallionen oder mit mindestens zwei basischen organischen Aminen bis zu einem optischen Reinheitsgrad (O.R.G.) von höher als 99%, ausgehend von einem racemischen Gemisch derselben, umfassend die folgenden Stufen:
a) Herstellung der Diaminosalze des genannten racemischen Gemisches durch Umsetzung von racemischer freier Folinsäure, gelöst in einem dipolaren aprotischen organischen Lösungsmittel oder in einem Gemisch desselben mit einem protischen organischen Lösungsmittel oder suspendiert in Wasser, mit den Aminen nach Anspruch 1, oder Herstellung der genannten Diaminosalze durch Umsetzung einer wäßrigen Lösung eines racemischen Erdalkalimetallfolinats mit einer Mineralsäure bei pH 1-3 bei 0-25°C und anschließend mit den genannten Aminen und Eliminierung der genannten Metallionen durch Bildung von unlöslichen Verbindungen derselben und Filtration derselben, wobei das erhaltene racemische Gemisch der genannten Diaminosalze aus dem Reaktionsmedium kristallisiert oder aus diesem durch Zugabe eines wasserlöslichen protischen organischen Lösungsmittels oder durch Verdünnung des genannten Reaktionsmediums in dem genannten wasserlöslichen protischen organischen Lösungsmittel ausgefällt wird,
b) Kristallisation des genannten ausgefällten racemischen Gemisches aus einer Lösung davon in einem Lösungsmittel aus Wasser und einem wasserlöslichen dipolaren aprotischen organischen Lösungsmittel, das gegebenenfalls ein protisches organisches Lösungsmittel enthält, wobei der sich bei der Kristallisation abscheidende Feststoff einen Hauptteil eines der zwei (6R)- oder (6S)-diastereomeren Formen des Diaminosalzes enthält, wobei diese Form weniger löslich ist als die andere, die zum großen Teil gelöst in den Kristallisations-Mutterlaugen zurückbleibt,
c) einmalige oder mehrmalige Umkristallisation des in Stufe b) erhaltenen, an dem Isomeren angereicherten Feststoffs aus der gleichen Art von Lösungsmittel wie in dieser Stufe verwendet, um das angestrebte (6R)- und (6S)-Folinat mit einem O.R.G. von höher als 99% zu erhalten, wobei das genannte optisch reine Diaminofolinat danach in die entsprechenden Erdalkalimetallsalze der gleichen optischen Reinheit durch Umsetzung mit einem Überschuß von wasserlöslichen Erdalkalimetall-Mineralsalzen in einem wäßrigen Medium umgewandelt werden kann,
d) Ausfällung des anderen, an Isomeren angereicherten Diaminosalzes aus den in Stufe b) erhaltenen Kristallisations-Mutterlaugen durch Verdünnung mit einem dipolaren aprotischen Lösungsmittel und/oder einem protischen organischen Lösungsmittel, Sammeln des Feststoffs davon und einmalige oder mehrmalige Umkristallisation desselben bei den gleichen Bedingungen wie in Stufe c), wobei die genannte andere stereoisomere Form des Diaminofolinats mit einem O.R.G. von höher als 99% erhalten wird, wobei dann gegebenenfalls die entsprechenden Erdalkalimetallsalze mit der gleichen optischen Reinheit durch Umsetzung mit einem Überschuß von wasserlöslichen Erdalkalimetall-Mineralsalzen erhalten werden,
oder wobei die genannten Mutterlaugen der Stufe b) nach Eliminierung von irgendwelchem organischem Lösungsmittel mit einem Überschuß eines wasserlöslichen Erdalkalimetall-Mineralsalzes direkt behandelt werden, wobei die erhaltenen, an Isomeren angereicherten Erdalkalimetallfolinate einmal oder mehrmals aus Wasser umkristallisiert werden, um die entsprechenden Metallfolinate mit einem O.R.G. von höher als 99% zu erhalten.

6. Verfahren nach Anspruch 5, dadurch **gekennzeichnet,** daß während der Stufe a) die Umsetzung der racemischen Folinsäure mit den genannten Aminen in einem dipolaren aprotischen organischen Lösungsmittel oder in einem Gemisch desselben mit einem protischen organischen Lösungsmittel oder in Wasser in Suspensionsphase durch anschließende Verdünnung der resultierenden Lösung mit einem oder in einem wasserlöslichen organischen protischen Lösungsmittel durchgeführt wird.

7. Verfahren nach Anspruch 5, dadurch **gekennzeichnet,** daß während der Stufe a) die Umsetzung der genannten racemischen Erdalkalimetallfolinate mit den genannten Aminen in Wasser in Gegenwart von anorganischen oder organischen Anionen, die wasserunlösliche Derivate mit den genannten Erdalkalimetallkationen bilden, durchgeführt wird, wobei die resultierenden unlöslichen Verbindungen vorzugsweise Sulfate oder Oxalate sind.

8. Verfahren nach Anspruch 5, dadurch **gekennzeichnet,** daß die Kristallisationen in den Stufen b) und c) in einem Lösungsmittelgemisch aus Wasser und einem wasserlöslichen dipolaren aprotischen organischen Lösungsmittel durchgeführt werden, wobei der Gehalt der genannten Komponenten im Volumenverhältnis im Bereich von 1:0,5 (V/V) bis 1:20 (V/V) liegt, oder daß die genannten Kristallisationen in einem Lösungsmittel, enthaltend das genannte Lösungsmittelgemisch aus Wasser und wasserlöslichem dipolarem aprotischem organischem Lösungsmittel, unter weiterem Zusatz eines protischen organischen Lösungsmittels in Mengen von bis zu 70 Vol.-% durchgeführt werden.

9. Verfahren nach Anspruch 5, dadurch **gekennzeichnet,** daß das Gewicht/Volumen-Verhältnis zwischen den genannten racemischen Diaminofolinatsalzen und dem zur Kristallisation derselben verwendeten Lösungsmittelgemisch in Stufe b) im Bereich von 1:2 (G/V) bis 1:60 (G/V) liegt.

10. Verfahren nach Anspruch 5, dadurch **gekennzeichnet,** daß die Kristallisation der genannten Folinsäuresalze in den Stufen b) oder c) durch Auflösung derselben in den genannten Lösungsmittelgemischen bei Temperaturen im Bereich von 25 bis 70°C und anschließende allmähliche Abkühlung der resultierenden Lösung auf 25-0°C durchgeführt wird.

11. Verfahren nach Anspruch 5, dadurch **gekennzeichnet,** daß die in Stufe c) erhaltenen, optisch reinen (6R) - oder (6S) -Diaminofolinate in die entsprechenden Erdalkalimetallsalze der gleichen optischen Reinheit in Wasser und in Gegenwart von wasserlöslichen Mineralsalzen der genannten Metalle bei einem Gewichtsverhältnis (G/G) im Bereich von 0,2 bis 5 Teilen des genannten Metallsalzes pro Teil des genannten Diaminosalzes bei einer Temperatur im Bereich von 0 bis 35°C durch Aufrechterhaltung des pH-Werts auf einen Wert zwischen 6 und 7, vorzugsweise durch Zugabe von 1N Natriumhydroxidlösung, umgewandelt werden.

12. Verfahren nach Anspruch 5, dadurch **gekennzeichnet,** daß die bevorzugten Kationen des Erdalkalimetalls aus Ca²⁺, Mg²⁺, Sr²⁺ und Ba²⁺ ausgewählt werden.

13. Verfahren nach Anspruch 12, dadurch **gekennzeichnet,** daß das bevorzugte Metallion Ca²⁺ ist.

14. Verfahren nach einem der Ansprüche 1 bis 13, dadurch **gekennzeichnet,** daß die bevorzugten Amine aus Ethylendiamin, 1,2-Diaminopropan, 1,3-Diaminopropan, 1,3-Diamino-2-hydroxypropan, (cis)-1,2-Diaminocyclohexan, (trans) -1,2-Diaminocyclohexan, Piperazin, 2-Methylpiperazin, 1,4-Dimethylpiperazin, 2,5-Dimethylpiperazin, 1,4,7,10-Tetraazacyclododecan sowie den Derivaten und den niedrigen und höheren Homologen davon ausgewählt werden.

15. Verfahren nach Anspruch 5, dadurch **gekennzeichnet,** daß die bevorzugten dipolaren aprotischen Lösungsmittel aus Dimethylformamid, Dimethylacetamid, Dimethylsulfoxid, N-Methylpyrrolidon und Hexamethylphosphoramid ausgewählt werden.

16. Verfahren nach Anspruch 5, dadurch **gekennzeichnet,** daß die bevorzugten protischen organischen Lösungsmittel aus Methanol, Ethanol, n-Propanol, Isopropanol, Ethylenglycol, 1,2-Propylenglycol, 1,3-Propylenglycol, Formamid und N-Methylformamid ausgewählt werden.

17. Verfahren nach Anspruch 5, dadurch **gekennzeichnet,** daß die in den Stufen c) oder d) erhaltenen optisch reinen Salze der Folinsäure in Wasser aufgelöst werden und daß die resultierenden wäßrigen Lösungen durch Zugabe einer Mineralsäure zu einem pH-Wert von 1-3 im Temperaturbereich von 0 bis 25°C angesäuert werden, wodurch die entsprechenden (6R)- oder (6S)-Isomeren der freien Folinsäure mit einem O.R.G. von höher als 99% ausfallen.

18. Verfahren nach Anspruch 11, dadurch **gekennzeichnet,** daß die genannten wasserlöslichen Mineralsalze der Erdalkalimetalle aus Chloriden und Nitraten ausgewählt werden.

19. Diamino-(6R,S) -Folinate, wobei das salzbildende Diamin aus Ethylendiamin, 1,2-Diaminopropan, 1,3-Diaminopropan, 1,3-Diamino-2-hydroxypropan, (cis)-1,2-Diaminocyclohexan, (trans)-1,2-Diaminocyclohexan, Piperazin, 2-Methylpiperazin, 1,4-Dimethylpiperazin und 2,5-Dimethylpiperazin ausgewählt worden ist.

20. Diamino-(6R)-Folinate mit einem O.R.G. von höher als 99%, wobei das salzbildende Diamin aus Ethylendiamin, 1,2-Diaminopropan, 1,3-Diaminopropan, 1,3-Diamino-2-hydroxypropan, (cis)-1,2-Diaminocyclohexan, (trans)-1,2-Diaminocyclohexan, Piperazin, 2-Methylpiperazin, 1,4-Dimethylpiperazin und 2,5-Dimethylpiperazin ausgewählt worden ist.

21. Diamino-(6S)-Folinate mit einem O.R.G. von höher als 99%, wobei das salzbildende Diamin aus Ethylendiamin, 1,2-Diaminopropan, 1,3-Diaminopropan, 1,3-Diamino-2-hydroxypropan, (cis)-1,2-Diaminocyclohexan, (trans)-1,2-Diaminocyclohexan, Piperazin, 2-Methylpiperazin, 1,4-Dimethylpiperazin und 2,5-Dimethylpiperazin ausgewählt worden ist.

22. Diaminofolinat, ausgewählt aus:
Ethylendiamino-(6R,S) -Folinat;
Piperazino-(6R,S)-Folinat;
1,4-Dimethylpiperazino-(6R,S)-Folinat;
1,3-Diamino-2-hydroxypropyl-(6R,S)-Folinat;
Ethylendiamino-(6R)-Folinat;
Ethylendiamino-(6S)-Folinat;
Piperazino-(6R)-Folinat;
Piperazino-(6S)-Folinat;
1,4-Dimethylpiperazino-(6R)-Folinat;
1,4-Dimethylpiperazino-(6S)-Folinat;
1,3-Diamino-2-hydroxypropyl-(6R)-Folinat; und
1,3-Diamino-2-hydroxypropyl-(6S)-Folinat.

## Revendications

1. Procédé pour la séparation des diastéréoisomères (6R) et (6S) de l'acide folinique et leur récupération sous forme libre ou sous forme de sel ayant une pureté optique élevée, ledit procédé comprenant les étapes consistant à :
a) faire réagir l'acide folinique racémique, éventuellement obtenu à partir de son sel, avec un métal alcalino-terreux par acidification avec des acides minéraux à un pH compris entre 1 et 3 à une température allant de 0 à 25 ° C, dans un milieu liquide contenant au moins un constituant choisi parmi des solvants organiques aprotiques dipolaires, de l'eau et des solvants organiques protiques hydrosolubles, avec une quantité stoechiométrique ou un excès molaire qui n'est pas supérieur à 20 % d'une diamine ayant la formule générale I à III 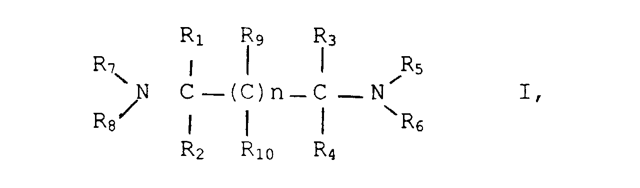 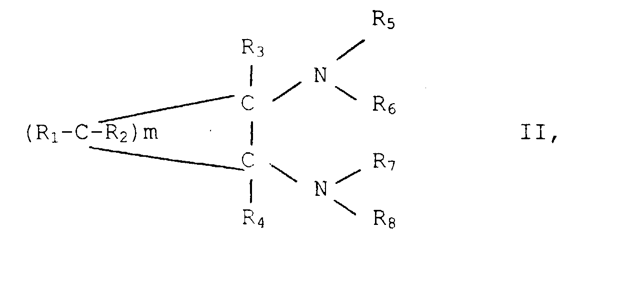 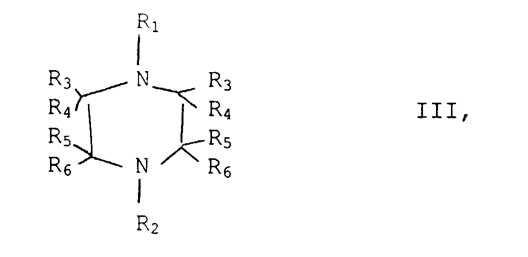 dans lesquelles :
R₁ à R₈ qui sont identiques ou différents, représentent H ou des groupes alkyles linéaires ou ramifiés en C₁₋₆, qui peuvent être substitués par 1 à 4 groupes OH et/ou par 1 à 4 groupes alcoxy ou hydroxyalcoxy,
R₉ à R₁₀ qui sont identiques ou différents, représentent R₁ ou OH,
n est compris entre 0 et 6
m est compris entre 2 et 8
ou ils sont choisis parmi des polyamines linéaires ou macrocycliques comprenant 3 à 6 atomes N pour donner le sel racémique folinique de di- ou de polyamines ;
b) à cristalliser ledit sel de diamine à partir de sa solution dans un mélange d'eau et d'un solvant organique aprotique dipolaire hydrosoluble, contenant éventuellement un solvant organique protique hydrosoluble, dans lequel un premier solide se séparant lors du refroidissement contient la majeure partie du premier desdits diastéréoisomères (6R) ou (6S), la majeure partie du second isomère restant dans les liqueurs-mères de cristallisation ;
c) à purifier en recristallisant à une ou plusieurs reprises le solide enrichi en isomère de l'étape b), à partir du même mélange de solvant que celui utilisé dans cette étape, jusqu'à ce que le premier sel de diamine diastéréoisomère soit obtenu avec la pureté optique souhaitée, et éventuellement à convertir ce sel en sel de métal alcalino-terreux correspondant ;
d) à isoler le solide demeuré dissous dans les liqueurs-mères de l'étape b), par précipitation en diluant la solution avec un solvant aprotique dipolaire et/ou un solvant organique protique, le solide qui est obtenu contenant essentiellement le second isomère,
et à recristalliser ce solide comme indiqué à l'étape c), ou à traiter lesdites liqueurs-mères, après évaporation des solvants organiques, avec un excès de sels minéraux de métal alcalino-terreux hydrosolubles et à recristalliser à une ou plusieurs reprises le sel de métal alcalino-terreux dudit second isomère d'acide folinique obtenu avec de l'eau jusqu'à ce que la pureté optique souhaitée soit atteinte.

2. Procédé selon la revendication 1, dans lequel on utilise dans l'étape a) un sel de métal alcalino-terreux d'acide folinique racémique, ledit cation de métal alcalino-terreux étant insolubilisé par réaction avec un anion approprié, puis éliminé par filtration.

3. Procédé selon la revendication 1, dans lequel le sel de diamine racémique d'acide folinique est isolé à l'étape a) par précipitation ou par cristallisation, en traitant le mélange réactionnel avec un solvant organique protique hydrosoluble.

4. Procédé selon la revendication 1, dans lequel le degré de pureté optique souhaité pour les deux isomères qui proviennent de la cristallisation des étapes c) ou d) est de 99 % ou plus.

5. Procédé selon la revendication 1 pour la séparation et la purification des deux formes diastéréoisomères, (6R) et (6S), de l'acide folinique et/ou de ses sels avec des ions métalliques alcalino-terreux ou avec au moins des amines organiques dibasiques, jusqu'à un degré de pureté optique (P.O.) supérieur à 99 %, à partir de leur mélange racémique, ce procédé comprenant les étapes consistant à :
a) préparer les sels de diamine dudit mélange racémique en faisant réagir l'acide folinique racémique libre, dissous dans un solvant organique aprotique dipolaire ou dans un mélange de ce dernier avec un solvant organique protique, ou mis en suspension dans l'eau, avec les amines de la revendication 1, ou à préparer lesdits sels de diamine en faisant réagir une solution aqueuse d'un folinate racémique de métal alcalino-terreux avec un acide minéral à un pH compris entre 1 et 3 à une température allant de 0 à 25 ° C, puis avec lesdites amines, et à éliminer lesdits ions métalliques par formation de leurs composés insolubles et leur filtration, le mélange racémique desdits sels de diamine obtenu cristallisant à partir du milieu réactionnel ou étant précipité à partir de ce milieu par addition d'un solvant organique protique hydrosoluble ou par dilution dudit milieu réactionnel dans ledit solvant organique protique hydrosoluble,
b) à cristalliser ledit mélange racémique précipité à partir d'une de ses solutions dans un solvant composé d'eau et d'un solvant organique aprotique dipolaire hydrosoluble contenant éventuellement un solvant organique protique, le solide qui se sépare lors de la cristallisation contenant la majeure partie de l'une des deux formes diastéréoisomères (6R) ou (6S) du sel de diamine, cette forme étant moins soluble que l'autre, qui reste largement dissoute dans les liqueurs-mères de cristallisation,
c) à recristalliser à une ou plusieurs reprises le solide enrichi en isomère obtenu à l'étape b), à partir du même type de solvant que celui utilisé dans cette étape, pour donner le folinate de diamine (6R) ou (6S) souhaité avec une P.O. supérieure à 99 %, ledit folinate de diamine optiquement pur pouvant ensuite être transformé en ses sels de métal alcalino-terreux correspondants avec la même pureté optique, par réaction avec un excès de sels minéraux de métal alcalino-terreux hydrosolubles dans un milieu aqueux,
d) à précipiter l'autre sel de diamine enrichi en isomère à partir des liqueurs-mères de cristallisation obtenu à l'étape b), par dilution avec un solvant aprotique dipolaire et/ou un solvant organique protique, à en recueillir le solide et à le recristalliser à une ou plusieurs reprises dans les mêmes conditions que dans l'étape c), ladite autre forme stéréoisomère du folinate de diamine étant obtenue avec une pureté optique supérieure à 99 %, les sels de métal alcalino-terreux correspondants étant ensuite éventuellement obtenus avec la même pureté optique par réaction avec un excès de sels minéraux de métal alcalino-terreux hydrosolubles,
ou à traiter directement lesdites liqueurs-mères de l'étape b), après élimination de tout solvant organique, avec un excès de sel minéral de métaux alcalino-terreux hydrosoluble, les folinates de métal alcalino-terreux enrichis en isomère obtenus étant recristallisés à une ou plusieurs reprises à partir de l'eau pour donner les folinates de métal correspondants avec une P.O. supérieure à 99 %.

6. Procédé selon la revendication 5, dans lequel, au cours de l'étape a), la réaction de l'acide folinique racémique avec lesdites amines est effectuée dans un solvant organique aprotique dipolaire, ou dans un de ses mélanges avec un solvant organique protique, ou dans de l'eau, en suspension, par dilution subséquente de la solution résultante avec ou dans un solvant protique organique hydrosoluble.

7. Procédé selon la revendication 5, dans lequel, au cours de l'étape a), la réaction desdits folinates racémiques de métal alcalino-terreux avec lesdites amines est effectuée dans de l'eau en présence d'anions organiques ou inorganiques, qui constituent des dérivés insolubles dans l'eau avec lesdits cations métalliques alcalino-terreux, lesdits composés insolubles résultants étant de préférence des sulfates ou des oxalates.

8. Procédé selon la revendication 5, dans lequel les cristallisations des étapes b) et c) sont effectuées dans un mélange de solvant composé d'eau et d'un solvant organique aprotique dipolaire hydrosoluble, lesdits constituants étant compris selon un rapport volumétrique de 1:0,5 (v/v) à 1:20 (v/v), ou lesdites cristallisations sont effectuées dans un solvant contenant ledit mélange eau/solvant organique aprotique dipolaire hydrosoluble auquel est ajouté en outre un solvant organique protique dans des quantités allant jusqu'à 70 % en volume.

9. Procédé selon la revendication 5, dans lequel le rapport poids/volume entre lesdits sels de folinate de diamine racémique et le mélange de solvant utilisé pour leur cristallisation selon l'étape b), se situe entre 1:2 (p/v) et 1:60 (p/v).

10. Procédé selon la revendication 5, dans lequel la cristallisation desdits sels d'acide folinique est effectuée selon les étapes b) ou c), en les dissolvant dans lesdits mélanges de solvant à une température allant de 25 à 70 ° C, puis en refroidissant progressivement la solution résultante jusqu'à 25-0 ° C.

11. Procédé selon la revendication 5, dans lequel les folinates de diamine optiquement purs (6R) ou (6S), obtenus dans l'étape c), sont transformés en leurs sels de métal alcalino-terreux correspondants ayant la même pureté optique dans l'eau, en présence de sels minéraux hydrosolubles desdits métaux, dans un rapport en poids (p/p) allant de 0,2 à 5 parties dudit sel de métal par partie dudit sel de diamine, à une température allant de 0 à 35 ° C, en maintenant le pH entre 6 et 7, de préférence en ajoutant de l'hydroxyde de sodium 1 N.

12. Procédé selon la revendication 5, dans lequel les cations de métal alcalino-terreux préférés sont choisis parmi Ca²⁺, Mg²⁺, Sr²⁺, Ba²⁺.

13. Procédé selon la revendication 12, dans lequel l'ion métallique préféré est Ca²⁺.

14. Procédé selon l'une des revendications 1 à 13, dans lequel les amines préférées sont choisies parmi l'éthylène diamine, le 1,2-diamino-propane, le 1,3-diamino-propane, le 1,3-diamino-2-hydroxy-propane, le (cis)-1,2-diamino-cyclohexane, le (trans)-1,2-diamino-cyclohexane, la pipérazine, la 2-méthylpipérazine, la 1,4-diméthyl-pipérazine, la 2,5-diméthyl-pipérazine, le 1,4,7,10-tétraazacyclododécane, leurs dérivés et leurs homologues inférieurs ou supérieurs.

15. Procédé selon la revendication 5, dans lequel les solvants aprotiques dipolaires préférés sont choisis parmi le diméthylformamide, le diméthylacétamide, le diméthylsulfoxyde, la N-méthylpyrrolidone, l'hexaméthylphosphoramide.

16. Procédé selon la revendication 5, dans lequel les solvants organiques protiques préférés sont choisis parmi le méthanol, l'éthanol, le n-propanol, l'isopropanol, l'éthylène glycol, le 1,2-propylène glycol, le 1,3-propylène glycol, le formamide, le N-méthyl-formamide.

17. Procédé selon la revendication 5, dans lequel les sels d'acide folinique optiquement purs, obtenus aux étapes c) ou d), sont dissous dans l'eau et les solutions aqueuses résultantes sont acidifiées par addition d'un acide minéral jusqu'à une valeur de pH comprise entre 1 et 3 à une température allant de 0 à 25 ° C, les isomères de l'acide folinique libre correspondants (6R) ou (6S) précipitant avec une P.O. supérieure à 99 %.

18. Procédé selon la revendication 11, dans lequel lesdits sels minéraux hydrosolubles de métaux alcalino-terreux sont choisis parmi des chlorures et des nitrates.

19. Folinates de diamine (6R,S) dans lesquels la diamine formant le sel est choisie parmi l'éthylène diamine, le 1,2-diamino-propane, le 1,3-diaminopropane, le 1,3-diamino-2-hydroxy-propane, le (cis)-1,2-diamino-cyclohexane, le (trans)-1,2-diaminocyclohexane, la pipérazine, la 2-méthyl-pipérazine, la 1,4-diméthyl-pipérazine, la 2,5-diméthyl-pipérazine.

20. Folinates de diamine (6R) avec une P.O. supérieure à 99 %, dans lesquels la diamine formant le sel est choisie parmi l'éthylène diamine, le 1,2-diamino-propane, le 1,3-diamino-propane, le 1,3-diamino-2-hydroxy-propane, le (cis)-1,2-diaminocyclohexane, le (trans)-1,2-diamino-cyclohexane, la pipérazine, la 2-méthyl-pipérazine, la 1,4-diméthylpipérazine, la 2,5-diméthyl-pipérazine.

21. Folinates de diamine (6S) avec une P.O. supérieure à 99 %, dans lesquels la diamine formant le sel est choisie parmi l'éthylène diamine, le 1,2-diamino-propane, le 1,3-diamino-propane, le 1,3-diamino-2-hydroxy-propane, le (cis)-1,2-diaminocyclohexane, le (trans)-1,2-diamino-cyclohexane, la pipérazine, la 2-méthyl-pipérazine, la 1,4-diméthylpipérazine, la 2,5-diméthyl-pipérazine.

22. Folinate de diamine choisi parmi :
le folinate d'éthylène diamine (6R, S) ;
le folinate de pipérazine (6R, S) ;
le folinate de 1,4-diméthyl-pipérazine (6R, S) ;
le folinate de 1,3-diamino-2-hydroxy-propyle (6R, S) ;
le folinate d'éthylène diamine (6R) ;
le folinate d'éthylène diamine (6S) ;
le folinate de pipérazine (6R) ;
le folinate de pipérazine (6S) ;
le folinate de 1,4-diméthyl-pipérazine (6R) ;
le folinate de 1,4-diméthyl-pipérazine (6S) ;
le folinate de 1,3-diamino-2-hydroxy-propyle (6R) ;
le folinate de 1,3-diamino-2-hydroxy-propyle (6S).
